# DEMANDE DE BREVET EUROPEEN

(11) **EP 3 127 504 A1**
(43) Date de publication de la demande: **08.02.2017**
(21) Numéro de dépôt: 16182487.5
(22) Date de dépôt: 03.08.2016
(51) Int. Cl.: A61B 90/70, A47L 15/48, G05D 7/01

(54) **DISPOSITIF DE LAVAGE ET DE SÉCHAGE D'ARTICLES**

(30) Priorité: 03.08.2015 FR 1557462
(71) Demandeur: Steris, 33185 Le Haillan (FR)
(72) Inventeur: VATELOT, Charles, 33470 Le Teich (FR); CHOROSY, Damien, 33290 Blanquefort (FR); OGER, Yvan, 33600 Pessac (FR)
(74) Mandataire: Robert, Mathias

(57) **Abrégé**

L'invention concerne un dispositif de lavage et de séchage d'articles comportant une cuve de lavage (1) dans laquelle les articles à laver sont destinés à être insérés ladite cuve (1) comportant au moins une paroi latérale (5, 6), et des moyens de séchage des articles, caractérisé en ce que lesdits moyens de séchage comportent des moyens d'amenée d'air débouchant à une première extrémité en partie supérieure de la paroi latérale (5) de la cuve (1), ladite première extrémité étant équipée d'un premier clapet (30), et à une seconde extrémité en partie inférieure de la paroi latérale (5) de la cuve (1), ladite seconde extrémité étant équipée d'un second clapet (32).

## Description

La présente invention concerne un dispositif de lavage et de séchage d'articles, tels que des instruments ou dispositifs médicaux, dentaires, pharmaceutiques ou vétérinaires.

De tels articles sont exposés au sang ou à des fluides corporels nécessitant leur nettoyage et/ou leur désinfection. Pour cela, il est connu d'utiliser des dispositifs de lavage permettant de traiter ces instruments ou dispositifs après chaque utilisation.

Les dispositifs de lavage permettent classiquement de réaliser une étape de lavage, une étape de rinçage et une étape de séchage des articles.

Le document WO 2009/058946 divulgue un dispositif de lavage de ce type, comportant un bâti comprenant une cuve de lavage dans laquelle les articles à laver sont destinés à être insérés.

La cuve comporte des parois latérales, une paroi inférieure et une paroi supérieure délimitant un espace supérieur avec une paroi du bâti. Le dispositif comporte une arrivée d'air externe au dispositif, débouchant dans l'espace supérieur, et équipée d'un filtre et d'un clapet unidirectionnel autorisant uniquement l'arrivée d'air extérieur (air de renouvellement) dans l'espace supérieur.

La paroi supérieure comporte un orifice d'aspiration d'air en regard duquel est disposée une turbine, logée dans l'espace supérieur.

En fonctionnement et lors d'une phase de séchage, la turbine aspire de l'air issu de la cuve, au travers de l'orifice précité et le rejette dans la cuve, soit en partie supérieure au travers de fentes ménagées entre les parois latérales et la paroi supérieure de la cuve, soit au travers de canalisations et de balais tournants montés en partie supérieure et en partie inférieure de la cuve. Parallèlement, un débit modéré d'air de renouvellement est prélevé à l'extérieur et pénètre dans l'espace supérieur. L'air circulant dans l'espace supérieur est chauffé à l'aide d'une résistance électrique. Par ailleurs, un évent débouchant au niveau de la paroi supérieure de la cuve permet de rejeter une partie de l'air de la cuve à l'extérieur du dispositif.

Il existe un besoin d'améliorer encore la qualité et la rapidité du séchage d'un tel dispositif. Ce dispositif nécessite également l'utilisation d'une turbine de grande puissance, servant à la fois à aspirer et à faire circuler de l'air de renouvellement et de l'air recyclé, c'est-à-dire issu de la cuve. Une telle turbine est onéreuse et présente une masse et un encombrement importants.

Afin de remédier à cet inconvénient, la demande de brevet FR 3 008 299 au nom de la Demanderesse propose un dispositif de lavage et de séchage d'articles, comportant un bâti comprenant une cuve de lavage dans laquelle les articles à laver sont destinés à être insérés, et des moyens de séchage des articles comportant des moyens de recyclage de l'air présent dans la cuve et des moyens de renouvellement de l'air conçus pour prélever de l'air extérieur au dispositif et le rejeter dans la cuve. Le dispositif comporte en outre un évent permettant de rejeter une partie de l'air hors du dispositif.

La cuve comporte des parois latérales délimitant, avec des parois du bâti, des canaux de circulation d'air de section oblongue et débouchant en partie basse de la cuve, des moyens d'aspiration de l'air situé en partie haute de la cuve étant conçus pour alimenter en air lesdits canaux. Les débouchés des canaux s'étendent sensiblement sur toute la largeur de la cuve.

De cette manière, l'air rejeté en partie basse de la cuve à l'aide des canaux de circulation est uniformément réparti sur la largeur de la cuve, puis traverse la cuve en séchant les articles qu'elle contient. L'air issu du canal de circulation circule dans la cuve de bas en haut, et est expulsé du canal de circulation de façon répartie sur la largeur de la cuve. Ceci permet d'améliorer sensiblement l'uniformité et les performances du séchage.

Les moyens de renouvellement de l'air et l'évent sont nécessaires afin de maintenir un taux d'humidité relativement faible au sein de la cuve et assurer ainsi un bon séchage des articles.

L'eau souillée présente dans la cuve peut toutefois pénétrer dans les canaux précités et stagner dans des volumes morts, ce qui peut permettre le développement de bactéries.

L'invention a notamment pour but d'apporter une solution simple, efficace et économique à ce problème, tout en offrant un séchage efficace et rapide.

A cet effet, elle propose un dispositif de lavage et de séchage d'articles comportant une cuve de lavage dans laquelle les articles à laver sont destinés à être insérés, ladite cuve comportant au moins une paroi latérale, et des moyens de séchage des articles, caractérisé en ce que lesdits moyens de séchage comportent des moyens d'amenée d'air débouchant :
- à une première extrémité en partie inférieure de la paroi latérale de la cuve, ladite première extrémité étant équipée d'un premier clapet apte à être ouvert si la pression de l'air en amont du premier clapet est supérieure à un seuil et apte à être fermé si ladite pression est inférieure audit seuil,
- à une seconde extrémité en partie supérieure de la paroi latérale de la cuve, ladite seconde extrémité étant équipée d'un second clapet apte à être ouvert si la pression de l'air en amont du second clapet est supérieure à un seuil et apte à être fermé si ladite pression est inférieure audit seuil.

L'utilisation de clapets permet d'éviter que, lors d'un cycle de lavage, de l'eau souillée pénètre dans les moyens d'amenée d'air, l'accès étant empêché par fermeture des premier et second clapets. On évite ainsi la stagnation d'eau dans des volumes mort ainsi que le développement de bactéries.

L'utilisation d'un clapet en partie inférieure et d'un clapet en partie supérieure permet de créer une circulation d'air dans la cuve, de façon à sécher rapidement et efficacement les articles qu'elle contient.

Le premier clapet peut être configuré pour générer un premier flux d'air entrant dans la cuve qui présente une forme annulaire tronconique d'axe perpendiculaire à la paroi latérale.

Le second clapet peut être configuré pour générer un second flux d'air entrant dans la cuve qui présente une forme annulaire et qui est orienté sensiblement parallèlement à la paroi latérale.

Il s'avère que, de façon surprenante, de tels flux d'air et leur interaction entre eux permettent de créer un flux d'air tourbillonnant dans la cuve, avec pour effet de sécher très rapidement les articles.

Le premier clapet et/ou le second clapet peuvent comporter un organe mobile entre une position de fermeture dudit clapet dans laquelle ledit organe mobile repose de façon étanche sur un siège dudit clapet, et une position d'ouverture dudit clapet dans laquelle l'organe mobile est écarté dudit siège, de façon à autoriser le passage d'air dans la cuve.

Dans ce cas, l'organe mobile du premier clapet peut comporter une surface d'appui tronconique, apte à venir en appui de façon étanche sur une surface complémentaire tronconique du siège.

Dans ce cas également, l'angle des surfaces tronconiques d'appui de l'organe mobile et du siège est compris entre 30 et 50°, de préférence de l'ordre de 45°, par rapport à l'axe desdites surfaces.

Par ailleurs, l'organe mobile du second clapet peut comporter une surface d'appui s'étendant radialement par rapport à l'axe de déplacement de l'organe mobile, apte à venir en appui de façon étanche sur une surface radiale complémentaire du siège.

En outre, l'organe mobile peut comporter au moins une partie en matériau souple, par exemple en silicone, comprenant au moins une surface d'appui apte à venir en appui de façon étanche sur une surface complémentaire du siège.

L'utilisation d'un matériau souple permet d'assurer une bonne étanchéité tout en limitant les chocs et les vibrations en fonctionnement, en vue de réduire les bruits et éviter une dégradation prématurée des différents éléments des clapets. L'utilisation de silicone par exemple permet également de résister à des températures élevées.

Le premier clapet et/ou le second clapet peuvent comporter des moyens de rappel élastiques de l'organe mobile dans sa position fermée. Les moyens de rappel élastiques sont par exemple formés par un ressort de compression hélicoïdal.

L'invention sera mieux comprise et d'autres détails, caractéristiques et avantages de l'invention apparaîtront à la lecture de la description suivante faite à titre d'exemple non limitatif en référence aux dessins annexés dans lesquels :
- la figure 1 est une vue schématique d'un dispositif selon l'invention,
- la figure 2 est une vue en coupe du premier clapet, dans sa position fermée,
- la figure 3 est une vue en coupe du second clapet, dans sa position fermée.

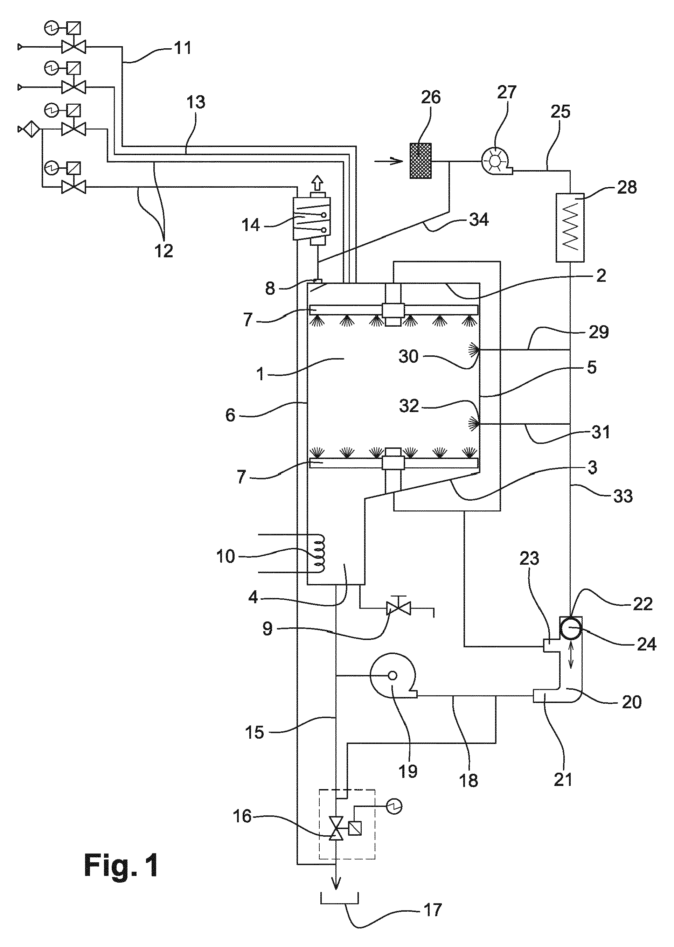

La figure 1 représente schématiquement un dispositif de lavage et de séchage d'articles selon une forme de réalisation de l'invention. Celui-ci comporte une cuve 1 délimitée par une paroi supérieure 2, une paroi inférieure 3 au niveau de laquelle est ménagé un réservoir 4, deux parois latérales fixes 5, 6 et deux parois latérales mobiles par exemple (non représentées). Les parois mobiles peuvent être formées par des portes coulissantes d'entrée et de sortie des articles.

La cuve 1 est équipée de balais 7 tournants d'aspersion montés au niveau des parois supérieure 2 et inférieure 3 de la cuve 1. Chaque balai 7 comporte des buses permettant le passage d'eau, lors d'une étape de lavage ou de rinçage par exemple, ou encore le passage d'air, lors d'une étape de séchage par exemple.

La cuve 1 est également pourvue d'un évent 8 en partie supérieure. Le réservoir 4 est équipé de moyens de purge 9 et de moyens de chauffage 10 comportant par exemple une résistance électrique ou un échangeur de chaleur apte à échanger de la chaleur entre l'eau présente dans le réservoir et un fluide caloporteur ou un fluide frigorigène.

Le dispositif comporte en outre des moyens d'amenée d'eau comportant notamment une conduite d'eau chaude 11, une conduite d'eau froide 12 et une conduite d'eau dite pure 13, c'est-à-dire ayant fait l'objet d'un traitement particulier en vue de réduire le nombre de bactérie présent dans l'eau ainsi que ses sels minéraux et polluants.

L'eau froide circulant dans la conduite 12 est apte à échanger de la chaleur avec de l'air issue de l'évent 8, par l'intermédiaire d'un échangeur de chaleur 14, de façon à refroidir cet air.

Par ailleurs, le dispositif comporte des moyens de recirculation de l'eau contenue dans le réservoir 4, ces moyens de recirculation comportant une conduite de recirculation comprenant une branche 15 équipée d'une vanne commandée 16 et débouchant au niveau d'un drain 17 et une branche 18 équipée d'une pompe de recirculation 19 et alimentant les balais d'aspersion 7. Un clapet 20 est situé entre la pompe de recirculation 19 et les balais 7. Ce clapet 20 comporte une première entrée 21 reliée à la pompe 19, une seconde entrée 22, et une sortie 23 reliée aux balais 7. Le clapet 20 comporte en outre un organe mobile 24 apte à restreindre le passage de la première entrée 21 ou obturer la seconde entrée 22 en fonction de la présence ou non de la pression de l'eau dans la branche 18.

Le dispositif comporte en outre des moyens d'amenée d'air à l'intérieur de la cuve 1, lesdits moyens d'air comportant une conduite d'amenée d'air 25 équipée d'un filtre 26, d'un compresseur ou ventilateur 27, et de moyens de chauffage de l'air 28, tels par exemple qu'une résistance électrique. En aval des moyens de chauffage 28, la conduite d'amenée d'air 25 se divise en une branche 29 alimentant un premier clapet 30 situé en partie supérieure de la cuve 1, sur la paroi latérale fixe 5 de la cuve 1, en une branche 31 alimentant un second clapet 32 située en partie inférieure de la cuve 1, sur la même paroi latérale fixe 5 que le premier clapet 30, et en une branche 33 reliée à la seconde entrée 22 du clapet 20.

Une conduite de renouvellement d'air 34 relie également un point situé entre l'évent 8 et l'échangeur 14, d'une part, et un point situé entre le filtre 26 et le compresseur ou ventilateur 27, d'autre part.

Ainsi, une partie de l'air destiné à la cuve 1 est de l'air dit frais et puisé à l'extérieur à la cuve 1, tandis qu'une autre partie est de l'air de renouvellement, issu de la cuve 1 et extrait au travers de l'évent 8.

Lors d'une phase de lavage ou de rinçage des articles présents dans la cuve 1, de l'eau est amenée dans la cuve 1 et dans le réservoir 4 puis alimente les balais d'aspersion 7 au travers successivement de la branche 18 équipée de la pompe 19 et du clapet 20, l'eau circulant entre l'entrée 21 et la sortie 23 du clapet 20. Dans une telle phase de fonctionnement, l'organe 24 obture l'entrée 21 du clapet 20 de façon à éviter que de l'eau de lavage ou de rinçage vienne souiller les moyens d'amenée d'air.

Lors d'une phase de séchage des articles présents dans la cuve, la pompe 19 est arrêtée, de sorte que l'organe mobile 24 du clapet 20 retombe et restreint le passage de l'entrée 21 du clapet 20. Le compresseur ou le ventilateur 27 est également démarré et de l'air traverse successivement le filtre 26 ou l'évent 8, le compresseur ou le ventilateur 27, et les moyens de chauffage 28 avant de déboucher dans la cuve 1 pour partie au travers des premier et second clapets 30, 32 et pour partie au travers des balais d'aspersion 7, en passant au travers du clapet 20.

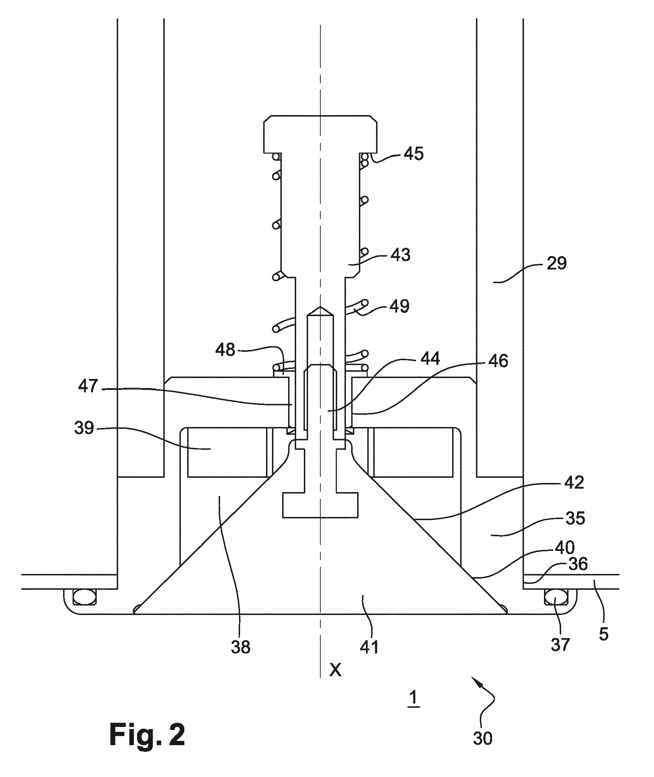

La structure du premier clapet 30, également appelé clapet supérieur, est représentée à la figure 2. Celui-ci comporte un corps fixe 35, monté dans un logement 36 de la paroi latérale correspondante 5 de la cuve 1, un joint torique 37 assurant l'étanchéité entre le corps 35 et ladite paroi 5. Le corps 35 comporte une chambre 38 présentant des ouvertures 39 permettant le passage d'air depuis la conduite correspondante 29, 31 vers la chambre 38. Le corps 35 comporte également une surface d'appui tronconique 40 tournée en direction de la cuve 1, ladite surface 40 formant un angle compris entre 30 et 50°, par exemple de l'ordre de 45°, avec l'axe X de ladite surface 40 et du corps 35. Un organe mobile 41, réalisé en matériau souple, par exemple en silicone, est monté dans la chambre 38 du corps 35. Ledit organe mobile 41 a une surface externe 42 tronconique tournée à l'opposé de la cuve 1 et de forme complémentaire à la surface 40 du corps 35. L'organe mobile 41 est apte à venir en appui de façon étanche sur la surface 40 du corps 35, en position fermée du clapet (représentée à la figure 2), cette surface 40 formant alors un siège.

L'organe mobile 41 est fixé à une tige d'actionnement 43 par l'intermédiaire d'une vis 44. La tige d'actionnement 43 s'étend selon l'axe X, est fixée à l'organe mobile 41 à une première extrémité et comporte un épaulement 45 tourné vers la cuve 1 à une seconde extrémité. La tige d'actionnement 43 traverse un trou 46 du corps 35, un palier lisse 47 d'axe X étant monté entre le corps 35 et la tige d'actionnement 43. Le palier 47 comporte une collerette 48 s'étendant radialement. Ladite tige d'actionnement 43 est soumise à l'action d'un organe élastique 49, tel par exemple qu'un ressort de compression hélicoïdal, ledit organe élastique 49 prenant appui sur le corps 35 ou sur la collerette 48 du palier 47, à une première extrémité, et sur l'épaulement 45 de la tige d'actionnement 43, à une autre extrémité. De cette manière, l'organe élastique 49 tend à rappeler l'organe mobile 41 en appui sur son siège 40.

Lorsque de l'air à une pression suffisante entre dans la chambre 38 par les ouvertures 39, la pression exercée sur l'organe mobile 41 tend à le déplacer en direction de la cuve 1, à l'encontre de l'effort de rappel exercé par l'organe élastique 49, de façon à décoller l'organe mobile 41 du siège 40 et former ainsi un passage annulaire permettant l'écoulement d'un flux d'air dans la cuve 1. Compte tenu de la forme du corps 35 et de l'organe mobile 41, ce flux d'air entrant dans la cuve 1 présente globalement une forme annulaire tronconique d'axe X perpendiculaire à la paroi latérale 5.

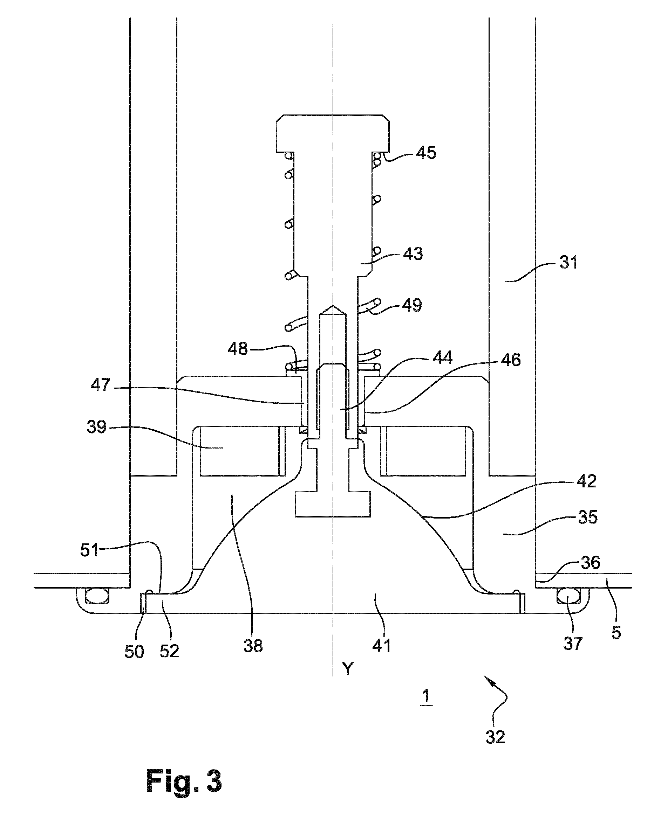

La structure du second clapet 32, également appelé clapet inférieur, est représenté à la figure 3. Celui-ci comporte, comme précédemment, un corps fixe 35, monté dans un logement 36 de la paroi latérale correspondante 5 de la cuve 1, un joint torique 37 assurant l'étanchéité entre le corps 35 et ladite paroi 5. Le corps 35 comporte une chambre 38 présentant des ouvertures 39 permettant le passage d'air depuis la conduite correspondante 31 vers la chambre 38. Le corps 35 comporte également un lamage 50 définissant une surface d'appui radiale 51, c'est-à-dire perpendiculaire à l'axe Y du corps 35, ladite surface radiale 51 étant tournée en direction de la cuve 1. Un organe mobile 41, réalisé en matériau souple, par exemple en silicone, est monté dans la chambre 38 du corps 35. Ledit organe mobile 41 a surface externe 42 globalement tronconique et légèrement bombée ou convexe, tournée à l'opposé de la cuve 1, prolongée au niveau de son extrémité tournée vers la cuve 1 par une collerette radiale annulaire 52. Ladite collerette annulaire 52 est apte à venir en appui de façon étanche sur la surface radiale 51 du corps 35, en position fermée du clapet 32, cette surface 51 formant alors un siège. En position fermée également, la collerette 52 est logée intégralement dans le lamage 50.

L'organe mobile 41 est fixé à une tige d'actionnement 43 par l'intermédiaire d'une vis 44. La tige d'actionnement 43 s'étend selon l'axe Y, est fixée à l'organe mobile 41 à une première extrémité et comporte un épaulement 45 tourné vers la cuve 1 à une seconde extrémité. La tige d'actionnement 43 traverse un trou 46 du corps 35, un palier lisse 47 d'axe Y étant monté entre le corps 35 et la tige d'actionnement 43. Le palier 47 comporte une collerette 48 s'étendant radialement. Ladite tige d'actionnement 43 est soumise à l'action d'un organe élastique 49, tel par exemple qu'un ressort de compression hélicoïdal, ledit organe élastique 49 prenant appui sur le corps 35 ou sur la collerette 48 du palier 47, à une première extrémité, et sur l'épaulement 45 de la tige d'actionnement 43, à une autre extrémité. De cette manière, l'organe élastique 49 tend à rappeler l'organe mobile 41 en appui sur son siège 51.

Lorsque de l'air à une pression suffisante entre dans la chambre par les ouvertures 39, la pression exercée sur l'organe mobile 41 tend à le déplacer en direction de la cuve 1, à l'encontre de l'effort de rappel exercé par l'organe élastique 49, de façon à décoller l'organe mobile 41 du siège 51 et former ainsi un passage annulaire permettant l'écoulement d'un flux d'air dans la cuve 1. Compte tenu de la forme du corps 35 et de l'organe mobile 41, ce flux d'air entrant dans la cuve 1 présente globalement une forme annulaire et est orienté sensiblement parallèlement à la paroi latérale 5.

Lors d'une phase de séchage, l'air sous pression amené par les conduites correspondantes 29, 31 décolle les organes mobiles 41 des clapets 30, 32 par rapport aux sièges correspondants 40, 51, de manière à ouvrir lesdits clapets 30, 32. Les flux d'air débouchant alors dans la cuve 1 par les clapets 30, 32 permettent de générer une circulation d'air supplémentaire dans la cuve 1, en plus des flux d'air issu des balais 7, en créant en particulier un flux d'air tourbillonnant dans la cuve 1, ce qui a pour effet d'améliorer l'efficacité du séchage et de réduire le temps d'un cycle de séchage, par comparaison avec l'art antérieur.

L'utilisation des clapets 30, 32 permet également d'éviter que, lors d'un cycle de lavage, de l'eau souillée pénètre dans les conduites d'amenée d'air 29, 31, l'accès étant empêché par la fermeture desdits clapets 30, 32. On évite ainsi la stagnation d'eau dans des volumes mort ainsi que le développement de bactéries.

## Revendications

1. Dispositif de lavage et de séchage d'articles comportant une cuve de lavage (1) dans laquelle les articles à laver sont destinés à être insérés ladite cuve (1) comportant au moins une paroi latérale (5, 6), et des moyens de séchage des articles, **caractérisé en ce que** lesdits moyens de séchage comportent des moyens d'amenée d'air débouchant :
- à une première extrémité en partie supérieure de la paroi latérale (5) de la cuve (1), ladite première extrémité étant équipée d'un premier clapet (30) apte à être ouvert si la pression de l'air en amont du premier clapet (30) est supérieure à un seuil et apte à être fermé si ladite pression est inférieure audit seuil,
- à une seconde extrémité en partie inférieure de la paroi latérale (5) de la cuve (1), ladite seconde extrémité étant équipée d'un second clapet (32) apte à être ouvert si la pression de l'air en amont du second clapet (32) est supérieure à un seuil et apte à être fermé si ladite pression est inférieure audit seuil.

2. Dispositif selon la revendication 1, **caractérisé en ce que** le premier clapet (30) est configuré pour générer un premier flux d'air entrant dans la cuve (1) qui présente une forme annulaire tronconique d'axe perpendiculaire à la paroi latérale (5).

3. Dispositif selon la revendication 1 ou 2, **caractérisé en ce que** le second clapet (32) est configuré pour générer un second flux d'air entrant dans la cuve (1) qui présente une forme annulaire et qui est orienté sensiblement parallèlement à la paroi latérale (5).

4. Dispositif selon l'une des revendications 1 à 3, **caractérisé en ce que** le premier clapet (30) et/ou le second clapet (32) comportent un organe (41) mobile entre une position de fermeture dudit clapet (30, 32) dans laquelle ledit organe mobile (41) repose de façon étanche sur un siège (40, 51) dudit clapet (30, 32), et une position d'ouverture dudit clapet (30, 32) dans laquelle l'organe mobile (41) est écarté dudit siège (40, 51), de façon à autoriser le passage d'air dans la cuve (1).

5. Dispositif selon la revendication 4, **caractérisé en ce que** l'organe mobile (41) du premier clapet (30) comporte une surface d'appui tronconique (42), apte à venir en appui de façon étanche sur une surface complémentaire tronconique (40) du siège.

6. Dispositif selon la revendication 5, **caractérisé en ce que** l'angle des surfaces tronconiques d'appui (42, 40) de l'organe mobile (41) et du siège est compris entre 30 et 50°, de préférence de l'ordre de 45°, par rapport à l'axe desdites surfaces (42, 40).

7. Dispositif selon l'une des revendications 4 à 6, **caractérisé en ce que** l'organe mobile (41) du second clapet (32) comporte une surface d'appui (52) s'étendant radialement par rapport à l'axe (Y) de déplacement de l'organe mobile (41), apte à venir en appui de façon étanche sur une surface radiale complémentaire (51) du siège.

8. Dispositif selon l'une des revendications 4 à 7, **caractérisé en ce que** l'organe mobile (41) comporte au moins une partie en matériau souple, par exemple en silicone, comprenant au moins une surface d'appui apte à venir en appui de façon étanche sur une surface complémentaire (40, 51) du siège.

9. Dispositif selon l'une des revendications 4 à 8, **caractérisé en ce que** le premier clapet (30) et/ou le second clapet (32) comportent des moyens (49) de rappel élastiques de l'organe mobile (41) dans sa position fermée.
